(19) Europäisches Patentamt
European Patent Office
Office européen des brevets

(11) Veröffentlichungsnummer: **0 306 869 B1**

(12) # EUROPÄISCHE PATENTSCHRIFT

(45) Veröffentlichungstag der Patentschrift: **18.09.91 Patentblatt 91/38**

(51) Int. Cl.[5]: **C07D 265/26**

(21) Anmeldenummer: **88114426.5**

(22) Anmeldetag: **03.09.88**

(54) **Verfahren zur Herstellung von Halogenisatosäureanhydriden.**

(30) Priorität: **11.09.87 DE 3730539**

(43) Veröffentlichungstag der Anmeldung: **15.03.89 Patentblatt 89/11**

(45) Bekanntmachung des Hinweises auf die Patenterteilung: **18.09.91 Patentblatt 91/38**

(84) Benannte Vertragsstaaten: **CH DE FR GB IT LI**

(56) Entgegenhaltungen:
**EP-A- 0 020 969**
**DE-A- 2 902 978**
**DE-A- 2 925 175**

(56) Entgegenhaltungen:
**CHEMICAL ABSTRACTS, Band 100, Nr. 25, 18. Juni 1984, Columbus, Ohio, USA; SUESSE, M.; JOHNE, S. "Quinazolinecarboxylic acids. 2. Quinazolin-4-on-2-ylacetamides" Seite 589, Spalte 1, Zusammenfassung-Nr. 209 731s**
**CHEMICAL ABSTRACTS, Band 101, Nr. 7, 13. August 1984, Columbus, Ohio, USA; SUESSE, M.; JOHNE, S. "Quinazolinecarboxylic acids. I. Quinazolin-4-one(2,4-dione)-3-acetic acids and esters" Seite 619, Spalte 1, Zusammenfassung-Nr. 55 043a**

(73) Patentinhaber: **BASF Aktiengesellschaft**
**Carl-Bosch-Strasse 38**
**W-6700 Ludwigshafen (DE)**

(72) Erfinder: **Kanter, Hartmut, Dr.**
**Niedererdstrasse 105**
**W-6700 Ludwigshafen (DE)**
Erfinder: **Kowarsch, Heinrich, Dr.**
**Kuernbacher Strasse 34**
**W-7519 Oberderdingen (DE)**
Erfinder: **Ort, Burkhard, Dr.**
**Waldstrasse 63**
**W-6706 Wachenheim (DE)**

EP 0 306 869 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die vorliegende Erfindung betrifft ein neues Verfahren zur Herstellung von Halogenisatosäureanhydriden durch Umsetzung von entsprechend unsubstituierten Isatosäureanhydriden mit elementarem Chlor oder Brom in Gegenwart von Chlorsulfonsäure.

Aus der DE-A-2206863 ist die Herstellung von 6-Chlorisatosäureanhydrid durch Umsetzung von Isatosäureanhydrid mit Sulfurylchlorid bekannt. Nachteilig bei dieser Verfahrensweise ist die Anwesenheit von inerten organischen Lösungsmitteln, wodurch die Raum-Zeit-Ausbeute beträchtlich herabgesetzt wird.

Aufgabe der vorliegenden Erfindung war es, ein neues Verfahren bereitzustellen, mittels dessen die Herstellung von Halogenisatosäureanhydriden in vorteilhafter Weise mit guter Raum-Zeit-Ausbeute und ohne großen technischen Aufwand gelingt.

Es wurde nun gefunden, daß die Herstellung von Halogenisatosäureanhydriden der Formel I

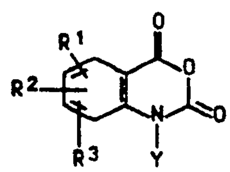

(I),

in der

Y Wasserstoff oder $C_1$-$C_4$-Alkyl und

$R^1$, $R^2$ und $R^3$ gleich oder verschieden sind und unabhängig voneinander jeweils Wasserstoff, Chlor, Brom, Nitro, $C_1$-$C_4$-Alkoxy, $C_1$-$C_4$-Alkyl, Trifluormethyl, $C_1$-$C_4$-Alkanoylamino, Carbamoyl, $C_1$-$C_4$-Mono- oder Dialkylcarbamoyl, Sulfamoyl oder $C_1$-$C_4$-Mono- oder Dialkylsulfamoyl bedeuten,

mit der Maßgabe, daß mindestens einer der Reste $R^1$, $R^2$ oder $R^3$ für Chlor oder Brom steht, durch Halogenierung von Isatosäureanhydriden der obengenannten Formel I, in der Y, $R^1$, $R^2$ und $R^3$ jeweils die obengenannte Bedeutung besitzen, wobei mindestens einer der Reste $R^1$, $R^2$ oder $R^3$ für Wasserstoff steht, vorteilhaft gelingt, wenn man die Halogenierung mit elementarem Chlor oder Brom in Gegenwart von Chlorsulfonsäure bei einer Temperatur von –10°C bis +100°C vornimmt und dabei je Mol Isatosäureanhydrid mindestens 0,5 Mol Halogen verwendet.

Alle in der obengenannten Formel I auftretenden Alkylgruppen können sowohl geradkettig als auch verzweigt sein.

$R^1$, $R^2$, $R^3$ und Y in Formel I bedeuten beispielsweise Methyl, Ethyl, Propyl, Isopropyl, Butyl, Isobutyl oder sec-Butyl.

$R^1$, $R^2$ und $R^3$ bedeuten weiterhin beispielsweise Methoxy, Ethoxy, Propoxy, Isopropoxy, Butoxy, Isobutoxy, sec-Butoxy, Formylamino, Acetylamino, Propionylamino, Butyrylamino, Isobutyrylamino, Mono- oder Dimethylcarbamoyl, Mono- oder Diethylcarbamoyl, Mono- oder Dipropylcarbamoyl, Mono- oder Diisopropylcarbamoyl, Mono- oder Dibutylcarbamoyl, N-Methyl-N-ethylcarbamoyl, Mono- oder Dimethylsulfamoyl, Mono- oder Diethylsulfamoyl, Mono- oder Dipropylsulfamoyl, Mono- oder Dibutylsulfamoyl oder N-Methyl-N-ethylsulfamoyl.

Vorzugsweise bedeuten Y Wasserstoff oder Methyl sowie $R^1$, $R^2$ und $R^3$ Wasserstoff, Chlor, Brom, oder Nitro.

Wenn man von kernunsubstituiertem Isatosäureanhydrid der Formel II

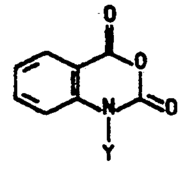

(II),

in der Y die obengenannte Bedeutung besitzt, ausgeht, gelingt mittels des erfindungsgemäßen Verfahrens die Herstellung sowohl von Mono- wie auch von Di- und Trihalogenisatosäureanhydriden.

Wenn man die Halogenierung in beliebiger Reihenfolge nacheinander mit Chlor und Brom vornimmt, gelangt man zu Halogenisatosäureanhydriden, die sowohl Chlor als auch Brom im aromatischen Kern aufweisen.

Erfindungsgemäß soll je Mol Isatosäureanhydrid I mindestens 0,5 Mol Halogen (Chlor oder Brom) verwendet werden.

Führt man eine Monohalogenierung durch, so verwendet man pro Mol Isatosäureanhydrid I vorzugsweise 0,5 bis 1,5 Mol Halogen. Für eine Di- oder Trihalogenierung sind diese Angaben in entsprechender Weise zu verdoppeln oder zu verdreifachen.

Es ist auch möglich, von bereits halogeniertem Isatosäureanhydrid auszugehen, wobei dann die Möglichkeit besteht, noch ein oder zwei Chlor- und/oder Bromatome in den aromatischen Kern einzuführen.

Vorzugsweise führt man das neue Verfahren in Gegenwart eines Katalysators durch. Als Katalysator dient Iod oder eine unter den Reaktionsbedingungen Iod freisetzende Verbindung.

Solche Verbindungen sind beispielsweise Iodwasserstoffsäure, Alkaliiodide, wie Natrium- oder Kaliumiodid, Alkaliiodate, wie Natrium- oder Kaliumiodat, oder Alkaliperiodate, wie Natrium- oder Kaliumperiodat.

Der Katalysator wird dabei in der Regel in einer Menge von 0,1 bis 2 Gew.%, bezogen auf das Lösungsmittel, zugegeben.

Als Lösungsmittel dient Chlorsulfonsäure, von der man im allgemeinen 2 bis 10 Gew.-Teile, bezogen auf 1 Gew.-Teil Isatosäureanhydrid, für die Umsetzung verwendet.

Eine bevorzugte Verfahrensweise besteht darin, die Halogenierung in einem Gemisch aus Chlorsulfonsäure und Oleum (20 bis 70 Gew.% an $SO_3$) durchzuführen. Dies ist vor allem dann von Bedeutung, wenn man Isatosäureanhydride herstellen will, die zwei oder drei Chlor- und/oder Bromatome im aromatischen Kern aufweisen.

Es hat sich dabei als besonders vorteilhaft erwiesen, der Chlorsulfonsäure 0,1 bis 2 Gew.-Teile Oleum, bezogen auf einen Gewichtsteil Isatosäureanhydrid I, zuzusetzen.

Erfindungsgemäß wird die Halogenierung bei einer Temperatur von −10°C bis +100°C, vorzugsweise 0 bis +50°C, vorgenommen.

Im allgemeinen wird das neue Verfahren unter Normaldruck vorgenommen. In manchen Fällen kann es jedoch von Vorteil sein, unter leicht erhöhtem Druck (bis zu 1,5 bar) zu arbeiten.

Das erfindungsgemäße Verfahren wird zweckmäßig so vorgenommen, daß man Chlorsulfonsäure, gegebenenfalls unter Zusatz von Oleum, vorlegt und bei einer Temperatur von −10 bis +50°C, vorzugsweise 0 bis +20°C, Isatosäureanhydrid und gegebenenfalls den Katalysator unter Rühren zugibt. Dann wird bei der genannten Temperatur das jeweilige Halogen zugegeben und die Temperatur gegebenenfalls erhöht.

Nach einer Nachrührphase von 1 bis 10 Stunden ist die Reaktion beendet. Das Reaktionsgemisch wird dann gegebenenfalls abgekühlt und auf 5 bis 20 Gew.-Teile Wasser, bezogen auf einen Teil Chlorsulfonsäure, gegeben. Das Reaktionsprodukt fällt dabei aus.

Beim Fällungsvorgang sollte die Temperatur bei 0 bis 30°C liegen. Dies wird durch Außenkühlung oder durch Fällung in Eiswasser erreicht.

Das Halogenisatosäureanhydrid wird dann abgetrennt, gewaschen und getrocknet.

Das neue Verfahren kann sowohl in kontinuierlicher als auch diskontinuierlicher Arbeitsweise vorgenommen werden.

Die Vorteile des erfindungsgemäßen Verfahrens liegen in seiner universellen Anwendbarkeit. Es werden hohe Raum-Zeit-Ausbeuten erzielt. Die Halogenierung erfolgt dabei in hoher Selektivität und ohne Ringöffnung. Weiterhin wird in Abwesenheit von organischen Lösungsmitteln gearbeitet.

Die mittels des erfindungsgemäßen Verfahrens erhaltenen Halogenisatosäureanhydride der Formel I sind wertvolle Zwischenprodukte für Farbstoffe, Pigmente und Pflanzenschutzmittel.

Die folgenden Beispiele sollen die Erfindung näher erläutern.

## Beispiel 1

60 g Isatosäureanhydrid wurden bei Raumtemperatur in 100 g Chlorsulfonsäure eingetragen. Nach Zugabe von 2 g Kaliumiodid wurden innerhalb von 3 Stunden 30 g Brom zugetropft und im Stickstoffstrom 10 Stunden nachgerührt. Das Reaktionsgemisch goß man dann langsam in 1200 g eines Eis/Wasser-Gemischs, ließ dabei die Temperatur nicht über 10°C ansteigen und saugte das ausgefällte Produkt ab. Man wusch mit wenig kaltem Wasser nach und trocknete bei 100°C. Man erhielt 75 g eines farblosen Pulvers (Brom-Wert 34,1%, theor. 33,1%) der Formel

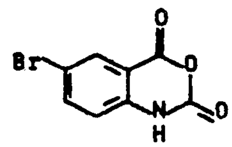

Beispiel 2

65 g Isatosäureanhydrid wurden bei Raumtemperatur in 120 g Chlorsulfonsäure eingetragen. Nach Zugabe von 2 g Kaliumiodid wurden innerhalb von 2,5 Stunden 48 g Brom zugetropft und 1 Stunde nachgerührt. Dann trug man innerhalb von 1 Stunde 47 g Oleum (65%ig) ein, ließ weitere 32 g Brom bei Raumtemperatur während 3 Stunden zutropfen und danach 2 Stunden nachrühren. Dann goß man das Reaktionsgemisch langsam in 1200 g Eis/Wasser-Gemisch, ließ dabei die Temperatur nicht über 10°C ansteigen und saugte das ausgefällte Produkt ab. Nach Waschen mit wenig kaltem Wasser und Trocknen bei 100°C erhielt man 128 g eines hellen Pulvers (Brom-Wert 49,6%, theor. 49,8%) der Formel

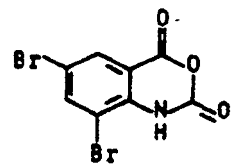

Beispiel 3

In 200 g Chlorsulfonsäure wurden bei 15 bis 20°C 70 g Isatosäureanhydrid und 2 g Kaliumiodid eingetragen. Dann wurden bei 20°C innerhalb 6 Stunden 40 g Chlor eingegast. Nach Zugabe von 120 g Oleum (65%ig) bei 15 bis 20°C wurden weitere 100 g Chlor bei 45 bis 50°C innerhalb von 6 Stunden eingegast und 2 Stunden bei dieser Temperatur nachgerührt. Die auf Raumtemperatur abgekühlte Lösung wurde danach auf 1200 g Eiswasser gegossen. Die Temperatur hielt man dabei durch Zugabe von Eis unter 10°C. Es wurde kalt abfiltriert, neutral gewaschen und getrocknet. Man erhielt 90 g eines farblosen Pulvers (Chlor-Wert 39,4%, theor. 39,9%) der Formel

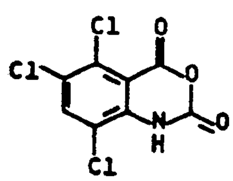

Beispiel 4

In 1000 g Chlorsulfonsäure wurden bei 5 bis 10°C 320 g Isatosäureanhydrid und 10 g kristallines Kaliumiodid eingetragen. Bei 15 bis 20°C wurden dann innerhalb 3 Stunden 170 g Chlor eingegast. Nach einstündigem Nachrühren gab man 260 g Oleum (65%ig) zu. Innerhalb von 3 Stunden tropfte man dann bei 15 bis 20°C 170 g Brom zu. Nach beendeter Zugabe wurde 2 Stunden auf 35 bis 40°C erwärmt. Die auf Raumtemperatur abgekühlte Lösung wurde auf eine Mischung aus 5000 g Eis und 2000 ml Wasser abgelassen und die Temperatur durch weitere Zugabe von 3000 g Eis auf 0°C gehalten. Es wurde kalt abfiltriert, mit kaltem Wasser neutral gewaschen und getrocknet. Man erhielt 460 g eines farblosen Pulvers (Chlor-Wert 12,4%, theor. 12,8%; Brom-Wert 29,3%, theor. 28,9%) der Formel

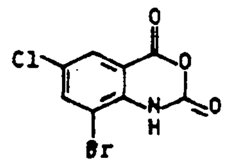

Beispiel 5

120 g 4-Chlor-isatosäureanhydrid und 2 g Kaliumiodid wurden in 440 g Chlorsulfonsäure vorgelegt. Dazu wurden bei Raumtemperatur innerhalb von 30 Minuten 210 g Brom zugetropft. Man ließ 5 Stunden nachrühren, erwärmte auf 50°C und rührte weitere 5 Stunden nach. Nach Abkühlen des Reaktionsgemisches goß man auf 2000 g Eis/Wasser-Gemisch, saugte das ausgefällte Produkt ab und trocknete es. Man erhielt 210 g eines hellen Pulvers (Chlor-Wert 9,7%, theor. 10,0% ; Brom-Wert 45,2%, theor. 45,0%) der Formel

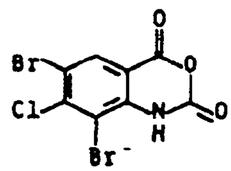

Die folgenden Beispiele wurden in analoger Weise durchgeführt.

Bsp.Nr. Reaktionsschema

6

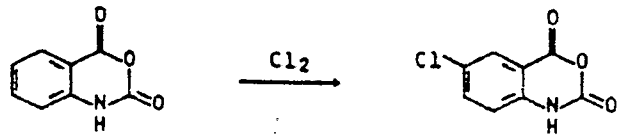


7

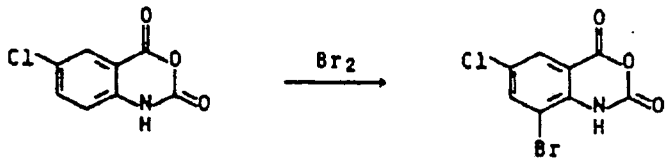


8

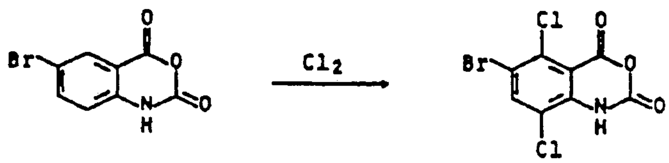


9

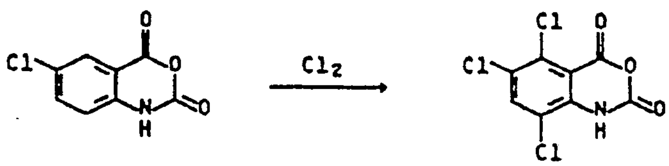


10

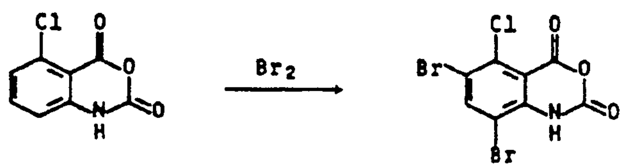


11

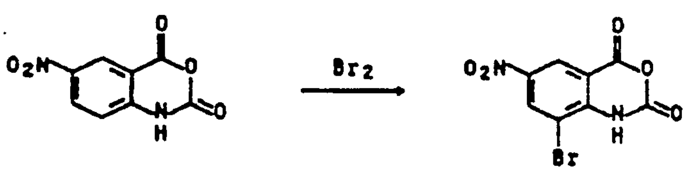


12

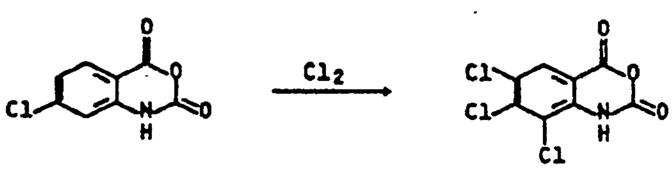


13

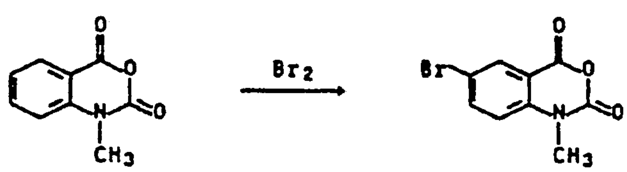

**Patentansprüche**

1. Verfahren zur Herstellung von Halogenisatosäureanhydriden der Formel I

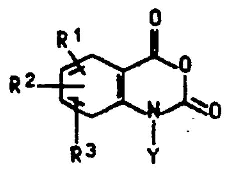

(I),

in der

Y Wasserstoff oder $C_1$-$C_4$-Alkyl und
$R^1$, $R^2$ und $R^3$ gleich oder verschieden sind und unabhängig voneinander jeweils Wasserstoff, Chlor, Brom, Nitro, $C_1$-$C_4$-Alkoxy, $C_1$-$C_4$-Alkyl, Trifluormethyl, $C_1$-$C_4$-Alkanoylamino, Carbamoyl, $C_1$-$C_4$-Mono-oder Dialkylcarbamoyl, Sulfamoyl oder $C_1$-$C_4$-Mono-oder Dialkylsulfamoyl bedeuten,

mit der Maßgabe, daß mindestens einer der Reste $R^1$, $R^2$ oder $R^3$ für Chlor oder Brom steht, durch Halogenierung von Isatosäureanhydriden der obengenannten Formel I, in der Y, $R^1$, $R^2$ und $R^3$ jeweils die obengenannte Bedeutung besitzen, wobei mindestens einer der Reste $R^1$, $R^2$ oder $R^3$ für Wasserstoff steht, dadurch gekennzeichnet, daß man die Halogenierung mit elementarem Chlor oder Brom in Gegenwart von Chlorsulfonsäure bei einer Temperatur von −10°C bis +100°C vornimmt und dabei je Mol Isatosäureanhydrid mindestens 0,5 Mol Halogen verwendet.

2. Verfahren gemäß Anspruch 1, dadurch gekennzeichnet, daß man die Halogenierung in Gegenwart von Iod oder einer unter den Reaktionsbedingungen Iod freisetzenden Verbindung als Katalysator vornimmt.

3. Verfahren gemäß Anspruch 1, dadurch gekennzeichnet, daß man die Halogenierung bei einer Temperatur von 0 bis +50°C vornimmt.

4. Verfahren gemäß Anspruch 1, dadurch gekennzeichnet, daß man 2 bis 10 Gew.-Teile Chlorsulfonsäure je Gew.-Teil Isatosäureanhydrid verwendet.

5. Verfahren gemäß Anspruch 1, dadurch gekennzeichnet, daß man die Umsetzung in einem Gemisch aus Chlorsulfonsäure und Oleum durchführt.

**Claims**

1. A process for preparing a haloisatoic anhydride of the formula I

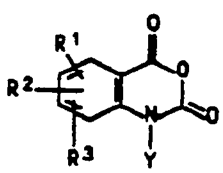

(I)

where

Y is hydrogen or $C_1$-$C_4$-alkyl and
$R^1$, $R^2$ and $R^3$ are identical or different and each is independently of the others hydrogen, chlorine, bromine, nitro, $C_1$-$C_4$-alkoxy, $C_1$-$C_4$-alkyl, trifluoro-methyl, $C_1$-$C_4$-alkanoylamino, carbamoyl, $C_1$-$C_4$-mono-alkyl- or -dialkyl-carbamoyl, sulfamoyl or $C_1$-$C_4$-monoalkyl- or -dialkyl-sulfamoyl,

with the proviso that at least one of the radicals $R^1$, $R^2$ or $R^3$ is chlorine or bromine, by halogenating an isatoic anhydride of the abovementioned formula I where Y, $R^1$, $R^2$ and $R^3$ are each as defined above and at least one of the radicals $R^1$, $R^2$ or $R^3$ is hydrogen with elemental chlorine or bromine in the presence of chlorosulfonic acid at from −10°C to +100°C

using not less than 0.5 mole of halogen per mole of isatoic anhydride.

2. A process as claimed in claim 1, wherein the halogenation is carried out in the presence of iodine, or of a compound which liberates iodine under the reaction conditions, as catalyst.

3. A process as claimed in claim 1, wherein the halogenation is carried out at from 0 to +50°C.

4. A process as claimed in claim 1, wherein from 2 to 10 parts by weight of chlorosulfonic acid are used per part by weight of isatoic anhydride.

5. A process as claimed in claim 1, wherein the reaction is carried out in a mixture of chlorosulfonic acid and oleum.

## Revendications

1. Procédé de préparation d'anhydrides isatoïques halogénés de formule I

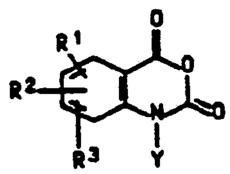

(I),

dans laquelle

Y est un atome d'hydrogène ou un reste alkyle en $C_1$-$C_4$,

$R^1$, $R^2$ et $R^3$ sont identiques ou différents et représentent chacun, indépendamment les uns des autres, un atome d'hydrogène, de chlore, de brome, un groupement nitro, $C_1$-$C_4$-alcoxy, $C_1$-$C_4$-alkyle, trifluorométhyle, $C_1$-$C_4$-alcanoylamino, carbamoyle, $C_1$-$C_4$-mono- ou dialkylcarbamoyle, sulfamoyle ou $C_1$-$C_4$-mono- ou dialkylsulfamoyle, étant spécifié que l'un au moins des restes $R^1$, $R^2$ ou $R^3$ est mis pour un atome de chlore ou de brome,

par halogénation d'anhydrides isatoïques répondant à la formule I ci-dessus dans laquelle Y, $R^1$, $R^2$ et $R^3$ ont respectivement les significations données précédemment, l'un au moins des restes $R^1$, $R^2$ ou $R^3$ étant mis pour un atome d'hydrogène, caractérisé en ce qu'on procède à l'halogénation avec du chlore ou du brome élémentaire en présence d'acide chlorosulfonique à une température de −10°C à +100°C, et on utilise au moins 0,5 mole d'halogène par mole d'anhydride isatoïque.

2. Procédé selon la revendication 1, caractérisé en ce qu'on procède à l'halogénation en présence, en tant que catalyseur, d'iode ou d'un composé qui libère de l'iode dans les conditions de la réaction.

3. Procédé selon la revendication 1, caractérisé en ce qu'on procède à l'halogénation à une température de 0 à +50°C.

4. Procédé selon la revendication 1, caractérisé en ce qu'on utilise de 2 à 10 parties en poids d'acide chlorosulfonique par partie en poids d'anhydride isatoïque.

5. Procédé selon la revendication 1, caractérisé en ce qu'on conduit la réaction dans un mélange d'acide chlorosulfonique et d'oléum.